(19) Europäisches Patentamt
European Patent Office
Office européen des brevets

(11) Publication number: 0 030 844 A1

(12) # EUROPEAN PATENT APPLICATION

(21) Application number: 80304472.6

(22) Date of filing: 11.12.80

(51) Int. Cl.³: C 07 C 103/52
C 12 P 21/04
//(C12P21/04, C12R1/66)

(30) Priority: 13.12.79 US 103014

(43) Date of publication of application: 24.06.81 Bulletin 81/25

(84) Designated Contracting States: AT BE CH DE FR GB IT LI LU NL SE

(71) Applicant: ELI LILLY AND COMPANY
307, East McCarty Street
Indianapolis Indiana 46206(US)

(72) Inventor: Michel, Karl Heinz
225, East North Street Apartment 2205
Indianapolis Indiana 46204(US)

(74) Representative: Crowther, Terence Roger et al,
Erl Wood Manor
Windlesham Surrey GU20 6PH(GB)

(54) **Recovery process for A-30912 antibiotics.**

(57) Process for recovery of A-30912-type antibiotics on a preparative scale which comprises purifying the antibiotic by reversed-phase liquid chromatography with a high-loading-capacity silica gel.

EP 0 030 844 A1

Croydon Printing Company Ltd

RECOVERY PROCESS FOR A-30912 ANTIBIOTICS

This invention relates to an improved process for the recovery of A-30912-type antibiotics. This process comprises purifying the antibiotic by reversed-phase liquid chromatography using a high-loading-capacity silica-gel adsorbent. The process is advantageous in that it can be used on a preparative scale and it provides a faster and more efficient isolation. Not only are higher yields obtained, but the materials isolated are in general of higher purity. A-30912-type antibiotics for which this process is advantageous include the following: the A-30912 antibiotics, the aculeacin antibiotics, and antibiotic S 31794/F-1.

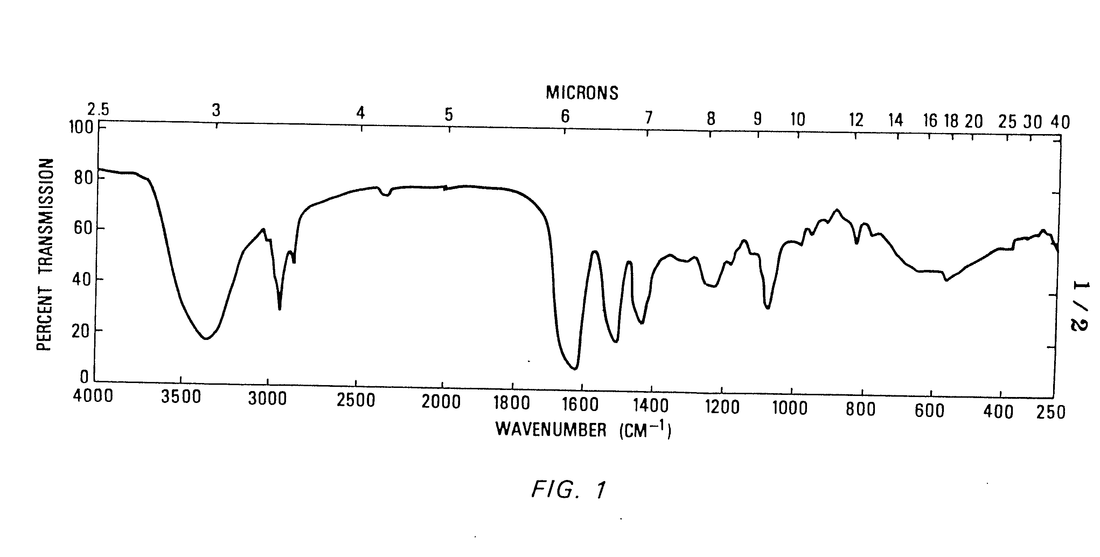

The infrared absorption spectrum of A-30912 factor H in KBr disc is presented in Figure 1 of the accompanying drawings.

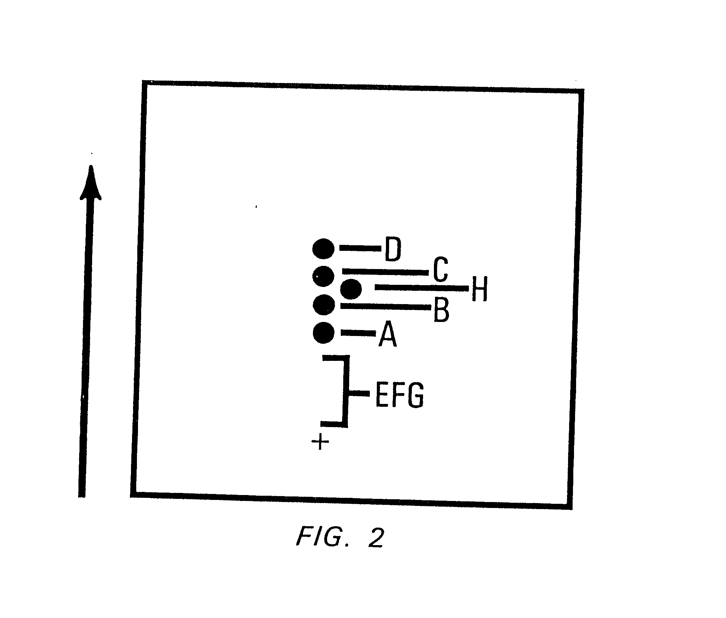

Figure 2 of the accompanying drawings summarizes the relative movements of the A-30912 factors on thin-layer chromatography (TLC), using high carbon content silica gel adsorbent, an ethyl acetate:methanol (3:2) solvent system, and *Candida albicans* bioautography for detection. The arrow indicates the direction of solvent flow; the "+" indicates the point of origin. The area where minor factors E, F, and G may be seen is indicated by bracketing. Specific $R_f$ comparisons between A-30912 factor A and minor factors E, F, and G are found in U.S. Patent 4,024,245 (see Table I in column 4).

This invention relates to an improved process of isolating certain antibiotics referred to herein as

A-30912-type antibiotics. This process comprises using reversed-phase liquid chromatography and a high-loading-capacity silica gel to purify the antibiotic by removing undesired components from the mixtures or complexes in which the antibiotic is obtained. Antibiotics of this type are generally produced as a mixture comprising several individual factors. The process of this invention is especially useful for separating the components of these mixtures.

The A-30912-type group of antibiotics for which the present invention is useful includes the A-30912 factors, the aculeacin factors, and antibiotic S 31794/F-1. This group of antibiotics is discussed in greater detail in the following paragraphs.

Specifically, this invention provides a process for recovering an A-30912-type antibiotic selected from the group consisting of A-30912 factors A, B, C, D, and H; the tetrahydro-derivatives of A-30912 factors A, B, C, D, and H; aculeacin factors A, Aα, Aγ, B, C, D, Dα, Dγ, E, F and G; and antibiotic S 31794/F-1; which comprises a) contacting a solution of said antibiotic with a reversed-phase high-loading-capacity silica gel, b) carrying out liquid chromatography and c) recovering the antibiotic.

The cultures which produce the antibiotics for which this process is useful are available from the stock culture collection of the Northern Regional Research Center, U.S. Department of Agriculture, Agricultural Research, Peoria, Illinois 61604. The accession number by which the culture may be obtained is the "NRRL" number.

## A. The A-30912 Factors

### 1. A-30912 Factor A

A-30912 factor A (antibiotic A-22082) is discussed in U.S. Patents 4,024,245 and 4,024,246. At the time these patents issued, it was believed that A-30912 factor A might be different from echinocandin B [see F. Benz *et al.*, *Helv. Chim. Acta 57*, 2459-2477 (1974) and Swiss Patent 568,386. Later, evidence has shown that A-30912 factor A is identical to echinocandin B. Antibiotic SL 7810/F has also been identified as echinocandin B [C. Keller-Juslen, *et al.*, *Tetrahedron Letters 1976* (46), 4147-4150, and Belgian Patent 834,289.

Keller-Juslen, *et al.*, proposed structure 1 for echinocandin B (SL 7810/F):

R = linoleoyl 1

Echinocandin B (antibiotic 32204) is produced by fermentation of a strain of *Aspergillus nidulans var. echinulatus* A 32204 (NRRL 3860), as discussed in Swiss patent No. 568,386. Antibiotic SL 7810/F is produced by a strain of *Aspergillus rugulosus*, Thom and Raper (NRRL 8039) as disclosed in Belgian Patent 834,289. Another method for producing A-30912 factor A by culturing *Aspergillus nidulans var. roseus* is the subject of a co-pending application No. 80301912.4 entitled "METHOD OF PRODUCING THE A-30912 ANTIBIOTICS". The antibiotic mixture produced by *A. nidulans var. roseus* has been called A-42355 antibiotic mixture.

For convenience, the A-30912 factor A designation will be used herein to refer to this antibiotic.

## 2. A-30912 Factors B and D

A-30912 factors B and D are described in U.S. Patent 4,024,245. A-30912 factor B is believed to be identical to antibiotic SL 7810/-II and to echinocandin C. A-30912 factor D is believed to be identical to antibiotic SL 7810/-III and to echinocandin D. A-30912 factors B and D (echinocandin C and D) have been shown to have structures 2 and 3, respectively [R. Traber, C. Keller-Juslen, H. Loosli, M. Kuhn and A. von Wartburg, *Helv. Chim. Acta 62* (4), 1252-1267 (1979):

R = linoleoyl

2: $R^1 = H$, $R^2 = R^3 = OH$ (A-30912 factor B)

3: $R^1 = R^2 = R^3 = H$ (A-30912 factor D)

Antibiotics SL 7810/-II and SL 7810/-III are produced by a strain of Aspergillus rugulosus, Thom and Raper (NRRL 8039) as disclosed in Belgian Patent 834,289.

3. A-30912 Factor H

A-30912 factor H (A-30912H) is a newly discovered component of the A-30912 antibiotic mixture. A-30912H is described in a co-pending application 80301913.2 entitled "A-30912 FACTOR H" Under conditions known thus far, A-30912H is a minor factor in the A-30912 mixture, being present in amounts in the range of from about 0.01 to about 1.0

percent of the total mixture. Another minor factor of the A-30912 mixture has been recognized, but has not been isolated in an amount sufficient for characterization. A-30912 factor H is best separated from this factor by silica-gel TLC using an ethyl acetate:methanol (3:2) or an acetonitrile:water (95:5) solvent system. In either system, the uncharacterized minor factor is more polar than the other A-30912 factors.

A-30912 factor H is a white amorphous solid. Elemental analysis of A-30912H gave the following approximate percentage composition: carbon, 58.27%; hydrogen, 7.49%; nitrogen, 8.67%; oxygen, 24.99%.

A-30912 factor H has a molecular weight of about 1073. This molecular weight is based upon peak matching (FD/FD) of the quasimolecular ions of A-30912 factor A ($C_{52}H_{81}N_7O_{16}$) and A-30912 factor H. The quasimolecular ion of A-30912 factor H (+Na) was found to have the mass 1096.5782, 1096.5814. The mean mass, 1096.5798, is within experimental error for $C_{53}H_{83}N_7O_{16}\cdot Na$, which has a theoretical mass of 1096.57940.

The approximate empirical formula of A-30912 factor H is, therefore, believed to be $C_{53}H_{83}N_7O_{16}$. The elemental analysis of A-30912 factor H corresponds especially well with the empirical formula of $C_{53}H_{83}N_7O_{16}\cdot H_2O$ (Calcd.: C, 58.30; H, 7.79; N, 8.98; O, 24.93).

The infrared absorption spectrum of A-30912 factor H in KBr disc is shown in Figure 1 of the accompanying drawings. The following characteristic absorption maxima are observed: 2.9 (very strong), 3.4

(strong), 3.5 (medium), 5.9-6.1 (very strong), 6.6 (strong), 6.9 (strong), 7.9-8.1 (medium), and 9.1 (strong) microns.

The ultraviolet absorption spectra of A-30912 factor H in both neutral and acidic methanol exhibit absorption maxima at 223 nm (ε 13,100) and 275 nm, broad peak (ε 2,100). The ultraviolet spectrum of A-30912 factor H in basic methanol shows absorption maxima at 245 nm (ε 14,700) and 290 nm, broad peak (ε 3,500), and also end absorption.

The $^{13}C$ nuclear magnetic resonance spectrum of A-30912 factor H in perdeuteromethanol shows the following characteristics:

δ 176.07, 174.15, 173.49, 172.56, 172.47, 169.88, 158.45, 133.01, 130.90, 129.52, 129.04, 116.21, 82.15, 77.00, 75.98, 75.71, 71.27, 69.55, 69.42, 68.22, 62.44, 58.69, 57.25, 56.81, 56.08, 52.88, 51.32, 39.08, 38.60, 36.89, 32.65, 30.77, 30.45, 30.26, 28.18, 27.14, 26.55, 20.11, 19.60, 11.33.

A-30912 factor H has the following approximate specific rotations:

$$[\alpha]^{25}_{D} \ -40° \ (\underline{c}\ 0.5,\ CH_3OH)$$

$$[\alpha]^{25}_{365} \ -151° \ (\underline{c}\ 0.5,\ CH_3OH)$$

Electrometric titration of A-30912 factor H in 66% aqueous dimethylformamide indicated the presence of a titratable group with a $pK_a$ value of about 12.90 (initial pH 7.12).

Amino acid analysis of A-30912 factor H indicated the presence, after hydrolysis, of threonine and four other as-yet-unidentified amino acids.

A-30912 factor H is soluble in a variety of organic solvents such as methanol, ethanol, dimethylformamide, dimethyl sulfoxide, and ethyl acetate; but is insoluble in nonpolar organic solvents such as diethyl ether and petroleum ether. A-30912 factor H is also soluble in aqueous solutions, especially those having a pH greater than 7.0.

A-30912 factor H (mol. wt. 1073) differs from A-30912 factor A (mol. wt. 1059) by only 14 mass units. Both compounds are very similar in physical-chemical properties, and both compounds produce linoleic acid upon hydrolysis. A-30912 factor H is, therefore, believed to be structurally related to A-30912 factor A. A-30912H appears to have an additional $-CH_2-$ which is present as $-O-CH_3$, replacing one of the -OH groups in the cyclic peptide portion of the molecule.

A-30912H is believed to have the structure shown in formula 4:

R = linoleoyl 4

The $R_f$ values of A-30912 factors A-G, using high carbon content silica gel TLC, a benzene:methanol (7:3) solvent system, and Candida albicans bioautography are given in Table I.

TABLE I

| A-30912 Factor | $R_f$ Value |
|---|---|
| A | 0.35 |
| B | 0.45 |
| C | 0.54 |
| D | 0.59 |
| E | 0.27 |
| F | 0.18 |
| G | 0.13 |

The approximate $R_f$ values of A-30912 factors A, B, C, D, and H in different solvent systems, using high carbon content silica gel TLC and *Candida albicans* bioautography, are given in Table II.

TABLE II

| A-30912 Factor | $R_f$ Values - Solvent Systems | | | |
|---|---|---|---|---|
| | a | b | c | d |
| Factor A | 0.28 | 0.14 | 0.28 | 0.43 |
| Factor B | 0.39 | 0.21 | 0.42 | 0.47 |
| Factor C | 0.46 | 0.31 | 0.51 | 0.58 |
| Factor D | 0.50 | 0.38 | 0.57 | 0.61 |
| Factor H | 0.42 | 0.27 | 0.36 | 0.53 |

Solvent Systems

a: ethyl acetate:methanol (3:2)

b: ethyl acetate:methanol (7:3)

c: acetonitrile:water (95:5)

d: ethyl acetate:ethanol:acetic acid (40:60:0.25)

4. The Tetrahydro-A-30912 Derivatives

A-30912 factors A, B, D and H each have a linoleoyl side chain. The tetrahydro-derivatives of A-30912 factors A, B, D, and H can be prepared by reducing the double bonds in this side chain using standard methods. The process of this invention is also useful for purification of these tetrahydro-derivatives, e.g. compounds of formulas 1 and 2 wherein R is stearoyl.

B. The Aculeacin Factors

The aculeacin mixture, comprising aculeacin A as a major factor and minor factors B, C, D, E, F, and G, is prepared as described by Mizuno *et al*. in U.S. Patent 3,978,210. The aculeacin mixture is produced by *Aspergillus aculeatus* M-4214 (FERM-P 2324), NRRL 8075. Aculeacin factors Aα, Aγ, Dα and Dγ are produced by *Aspergillus aculeatus* M 4845, NRRL 11270, as described in Belgian Patent 866,095.

Although there is no published information on the structure of the aculeacin antibiotics, it is recognized that they are analogous to the A-30912 antibiotics [see S. Satoi et al., *J. Antibiotics 30*, 303-307 (1977)]. The possibility that aculeacin A has the same cyclic peptide nucleus as tetrahydro-A-30912A is discussed in Belgian Patent 859,067. It is reported that palmitoyl is the fatty acid side chain of aculeacins A, Aγ, B, C, D, Dγ, E, F, and G and that myristoyl is the fatty acid side chain of aculeacins Aα and Dα.

Aculeacins Aα, Aγ, Dα and Dγ have the following properties:

| Analysis: | C(%) | H(%) | N(%) |
|---|---|---|---|
| Aα | 56.48 | 8.60 | 9.24 |
| Aγ | 54.70 | 8.23 | 10.51 |
| Dα | 55.96 | 8.44 | 9.96 |
| Dγ | 57.55 | 8.32 | 8.94 |

| | mol.wt. (Rast) | m.pt.(°C) | $[\alpha]_D^{23}$ (c=0.25,MeOH) |
|---|---|---|---|
| Aα | 1015 | 174-177 | -46.8° |
| Aγ | 1021 | 172-175 | -47.4° (c = 0.18) |
| Dα | 1243 | 159-162 | -45.7° |
| Dγ | 1265 | 159-162 | -46.5° |

Color Reactions:- positive to Pauli, Folin, $HIO_4$/benzidine and $KMnO_4$ decolorization reactions; weakly positive to Molisch, biuret, xanthoprotein and Tollens reactions; negative to ninhydrin, Benedict, Sakaguchi, Ehrlich, $FeCl_3$, Dragendorff and 2,4-dinitrophenylhydrazine reactions.

Aculeacins Aα, Aγ, Dα and Dγ are soluble in lower alcohols, sparingly soluble in ethyl acetate and are insoluble in acetone, $CHCl_3$, n-hexane, petroleum ether and water; are neutral (i.e. not transferred from n-butanol to water at pH 2-9) white and stable at 37°C. for 20 hours in acid and neutral conditions, but are unstable in alkaline conditions.

| | Liq. Phase Chromatography Retention Time (min.) | Fatty Acid Liberated in Hydrolysate |
|---|---|---|
| Aα | 7.9 | myristic |
| Aγ | 12.3 | palmitic |
| Dα | 9.2 | myristic |
| Dγ | 14.1 | palmitic |

The antibiotics are antifungal agents having activity against yeasts such as Candida albicans, dermatophytes such as Trichophyton rubrum, and plant-pathogenic fungi such as Diaporthe phaseolorum.

C. Antibiotic S 31794/F-1

Antibiotic S 31794/F-1, which is disclosed in German Offenlegungschrift 2,628,965 and in U.S. Patent 4,173,629, is an antifungal compound produced by Acrophialophora limonispora nov. spec. Dreyfuss et Muller NRRL 8095. S 31794/F-1 has the following characteristics: m.p. 178-180°C. (dec.) (amorphous) or 181-183°C. (dec.) (crystalline); $[\alpha]_D^{20}$ -24° (c 0.5, $CH_3OH$) or +37° (c 0.5, methanol) (crystalline); UV absorption maxima at 194 nm ($E_{1cm}^{1\%}$= 807), 225 nm (shoulder) ($E_{1cm}^{1\%}$= 132), 276 nm ($E_{1cm}^{1\%}$= 12.8), 284 nm (shoulder) ($E_{1cm}^{1\%}$= 10.5); is readily soluble in methanol, ethanol, pyridine, dimethyl sulfoxide and poorly soluble in water, chloroform, ethyl acetate, diethyl ether, benzene and hexane; and has antifungal activity, especially against Candida albicans.

Antibiotic S 31794/F-1 is believed to have structure 5, as follows:

R = myristoyl 5

The Recovery Process

The improved process of this invention comprises purifying A-30912-type antibiotics by reversed-phase liquid chromatography (LC) using a high carbon content silica gel absorbent. Such purification generally involves separating the component factors from the mixture in which they are produced. This process may also be used, however, for purification of the antibiotics and for purification of derivatives of the antibiotics, such as, for example, the tetrahydro-A-30912 derivatives.

Reversed-phase high performance liquid chromatography (HPLC) is a preferred method of this invention. Reversed-phase high performance, low pressure liquid chromatography (HPLPLC) is an especially preferred method.

Reversed-phase LC using a high carbon content silica gel may be used for large-scale preparative separations. It is more efficient and faster than previously used separation processes. In addition, the antibiotics obtained by this process are generally of improved purity.

The crude antibiotic mixture can be recovered from the fermentation medium in which it is produced by processes known in the fermentation art.

When using the process of this invention, a preliminary chromatographic separation using a standard absorbent may be used to remove gross impurities from the antibiotic mixture. Suitable absorbents for such an initial separation include silica gel; Florisil; Sephadex G-25, LH-20, and G-15; alumina; Diaion HP-20; and Amberlite XAD-4 and X-384. Diaion is available from Mitsubishi Chemical Industries, Tokyo; the Amberlite resins are available from Rohm and Haas Co., Philadelphia, Pa.; the Sephadex resins are available from Pharmacia Fine Chemicals AB, Uppsala, Sweden; and Florisil is available from Floridin Co., Tallahassee, Fla.

In the process of this invention the factors of the antibiotic mixture are rapidly and efficiently separated by LC using reversed-phase resins of high carbon content. High carbon content refers to a carbon content of 15 percent or above. Preferably, the carbon content is in the range of from about 19 to about 22 percent. Such reversed-phase resins have a high loading capacity. Two such resins include LiChroprep RP-8 and RP-18 which are available from E. Merck, Darmstadt, Germany.

In addition, a high loading capacity silica gel/$C_{18}$, prepared from Quantum LP-1 silica gel (Whatman), which has a carbon content of about 20 percent is described in Reference Example 2. The preparation involves silylation with octadecyltrichlorosilane and a second silylation to complete the high carbon loading. After the first silylation, the carbon content is in the range of from 10-14%. The second silylation increases the carbon content to 15 percent or above. The percentage of carbon achieved by the second silylation depends upon the amount of reagent used. Thus, using less reagent gives a lower carbon content. A carbon content of 15 percent or above is necessary to give the more efficient isolation of this invention.

In carrying out the process of this invention, the antibiotic to be purified is dissolved in an appropriate solvent, and the resulting solution is placed on a column equilibrated with the same or similar solvent. The column is then eluted with solvent. Generally, the same solvent or solvent system is used initially. Later the polarity of the solvent may be gradually changed to effect the required separation. The solvent or solvent system chosen will depend upon the solubility of the antibiotic. Those in the field will recognize that more than one solvent system may be used, but that certain solvent systems are preferred. For example, methanol:water:acetonitrile is a preferred solvent system for A-30912-type antibiotics, and methanol:water:-acetonitrile in a ratio of 7:2:1 is an especially preferred solvent system.

Fractions collected are conveniently monitored by bioautography against a sensitive microorganism and/or by UV (based on relative retention times). Fractions containing the desired factor are combined. It is sometimes necessary to carry out an additional chromatographic separation in order to obtain the antibiotic factor in especially purified form.

To illustrate the method of this invention, A-30912 factors A, B, D and H can be separated by reversed-phase HPLPLC using the following conditions:

| | |
|---|---|
| Column: | glass, 0.8 x 15.0 cm |
| Packing: | Nucleosil® 10-$C_{18}$ (Machery-Nagel and Company); packed using slurry-packing procedure of Reference Example 3. |
| Solvent: | methanol:water:acetonitrile (7:2:1) |
| Sample Volume: | 8 mcl |
| Sample Size: | 8 mcg |
| Column Temperature: | ambient |
| Flow Rate: | 1.8 ml/min |
| Pressure: | ca. 200 psi |
| Detector: | UV at 222 nm (ISCO Model 1800 variable Wavelength UV-Visible Absorbance Monitor) |
| Pump: | LDC Duplex Minipump |
| Injection: | loop injection |

The approximate retention times for A-30912 factors A, B, D, and H under these conditions are summarized in Table IV.

Table IV

| A-30912 Factor | Retention Time (seconds) |
|---|---|
| A | 792 |
| B | 870 |
| H | 990 |
| D | 1,140 |

In order to illustrate this invention even more fully, the following examples are provided.

Reference Example 1

Separation of the A-42355 Antibiotic Mixture

A. Shake-Flask Fermentation

A culture of *Aspergillus nidulans* var. *roseus* NRRL 11440 is prepared and maintained on an 18- x 150-ml agar slant prepared with medium having the following composition:

| Ingredient | Amount |
|---|---|
| Glucose | 5 g |
| Yeast extract | 2 g |
| $CaCO_3$ | 3 g |
| Vegetable juice* | 200 ml |
| Agar** | 20 g |
| Deionized water | q.s. to 1 liter |

(initial pH 6.1)

*V-8 Juice, Campbell Soup Co., Camden, N.J.

**Meer Corp.

The slant is inoculated with Aspergillus nidulans var. roseus NRRL 11440, and the inoculated slant is incubated at 25°C. for about seven days. The mature slant culture is covered with water and scraped with a sterile loop to loosen the spores. The resulting suspension is further suspended in 10 ml of sterile deionized water. One ml of the suspended slant growth is used to inoculate 55 ml of vegetative medium in a 250-ml flask. The vegetative medium has the following composition:

| Ingredient | Amount |
|---|---|
| Sucrose | 25 g |
| Blackstrap molasses | 36 g |
| Corn-steep liquor | 6 g |
| Malt extract | 10 g |
| $K_2HPO_4$ | 2 g |
| Enzymatic hydrolysate of casein* | 10 g |
| Tap water | 1100 ml |

(initial pH 6.5-6.7)

*N-Z-Case, Humko Sheffield Chemical, Lyndhurst, N.J.

The inoculated vegetative medium is incubated at 25°C. for 48 hours at 250 rpm on a rotary-type shaker. After 24 hours, the medium is homogenized for one minute at low speed in a blender (Waring type) and then returned to incubation for the remaining 24 hours. Alternatively, the inoculated vegetative medium can be incubated for 48 hours and then homogenized for 15 seconds at low speed.

This incubated vegetative medium may be used to inoculate shake-flask fermentation culture medium or to inoculate a second-stage vegetative medium. Alternatively, it can be stored for later use by maintaining the culture in the vapor phase of liquid nitrogen. The culture is prepared for such storage in multiple small vials as follows:
The vegetative cultures are mixed volume/volume with a suspending solution having the following composition:

| Ingredient | Amount |
|---|---|
| Glycerol | 20 ml |
| Lactose | 10 g |
| Deionized water | q.s. to 100 ml |

The prepared suspensions are distributed in small sterile screw-cap tubes (4 ml per tube). These tubes are stored in the vapor phase of liquid nitrogen.

A stored suspension thus prepared can be used to inoculate either agar slants or liquid seed media. Slants are incubated at 25°C. in the light for 7 days.

B. Tank Fermentation

In order to provide a larger volume of inoculum, 10 ml of incubated first-stage vegetative culture is used to inoculate 400 ml of a second-stage vegetative growth medium having the same composition as that of the vegetative medium. The second-stage medium is incubated in a two-liter wide-mouth Erlenmeyer flask at 25°C. for 24 hours on a shaker rotating through an arc two inches in diameter at 250 rpm.

Incubated second-stage medium (800 ml), prepared as above described, is used to inoculate 100 liters of sterile production medium selected from one of the following:

Medium I

| Ingredient | Amount |
|---|---|
| $ZnSO_4 \cdot 7H_2O$ | 0.00455 g/L |
| Soluble meat peptone* | 30.5 g/L |
| Soybean meal | 15.5 g/L |
| Tapioca dextrin** | 2.0 g/L |
| Blackstrap molasses | 10.5 g/L |
| Enzymatic hydrolysate of casein*** | 8.5 g/L |
| $Na_2HPO_4$ | 4.5 g/L |
| $MgSO_4 \cdot 7H_2O$ | 5.5 g/L |
| $FeSO_4 \cdot 7H_2O$ | 0.1 g/L |
| Cottonseed oil | 40.0 ml |
| (Antifoam)**** | 1.0 ml |
| Tap water | 1000.0 ml |

(initial pH 6.8-7.0)

*O.M. Peptone, Amber Laboratories, Juneau, Wisc.

**Stadex 11, A.E. Staley Co., Decatur, Ill.

***N-Z-Amine A, Humko Sheffield Chemical, Lyndhurst, N.J.

****P2000, Dow Corning, Midland, Michigan.

MEDIUM II

| Ingredient | Amount |
|---|---|
| Glucose | 2.5% |
| Starch | 1.0% |
| Soluble meat peptone* | 1.0% |
| Blackstrap molasses | 1.0% |
| $CaCO_3$ | 0.2% |
| $MgSO_4 \cdot 7H_2O$ | 0.05% |
| Enzymatic hydrolysate of casein** | 0.4% |
| (Antifoam)*** | 0.02% |
| Tap water | q.s. to volume |

*O.M. Peptone

**N-Z-Amine A

***Antifoam "A", Dow Corning

The inoculated production medium is allowed to ferment in a 165-liter fermentation tank at a temperature of 25°C. for about 7 days. The fermentation medium is aerated with sterile air, maintaining the dissolved oxygen level above approximately 50 percent of air saturation.

C. Third-Stage Vegetative Medium

Whenever the fermentation is carried out in tanks larger than those used for 100-liter fermentation, it is recommended that a third-stage vegetative culture be used to seed the larger tank. A preferred third-stage vegetative medium has the following composition:

| Ingredient | Amount |
|---|---|
| Sucrose | 25 g |
| Blackstrap molasses | 25 g |
| Corn-steep liquor | 6 g |
| Enzymatic hydrolysate of casein* | 10 g |
| Malt extract | 10 g |
| $K_2HPO_4$ | 2 g |
| Tap water | 1000 ml |

(initial pH 6.1)

*N-Z-Case

D. Separation of the A-42355 Antibiotic Mixture

Whole fermentation broth (4127 liters), obtained by the method described in Section B using Medium II, was stirred thoroughly with methanol (4280 liters) for one hour and then was filtered, using a filter aid (Hyflo Super-cel, a diatomaceous earth, Johns-Manville Products Corp.). The pH of the filtrate was adjusted to pH 4.0 by the addition of 5 N HCl. The acidified filtrate was extracted twice with equal volumes of chloroform. The chloroform extracts were combined and concentrated under vacuum to a volume of about 20 liters. This concentrate was added to about 200 liters of diethyl ether to precipitate the A-42355 mixture. The precipitate was separated by filtration to give 2775 g of the A-42355 mixture as a gray-white powder.

## Reference Example 2

## Preparation of Silica Gel/$C_{18}$ Reversed Phase Resin

### Step 1: Hydrolysis

LP-1 silica gel (1000 g from Quantum Corp., now Whatman) is added to a mixture of concentrated sulfuric acid (1650 ml) and concentrated nitric acid (1650 ml) in a 5-L round-bottom flask and shaken for proper suspension. The mixture is heated on a steam bath overnight (16 hours) with a water-jacketed condenser attached to the flask.

The mixture is cooled in an ice bath and carefully filtered using a sintered-glass funnel. The silica gel is washed with deionized water until the pH is neutral. The silica gel is then washed with acetone (4 L) and dried under vacuum at 100°C. for 2 days.

### Step 2: First Silylation

The dry silica gel from Step 1 is transferred to a round-bottom flask and suspended in toluene (3.5 L). The flask is heated on a steam bath for 2 hours to azeotrope off some residual water. Octadecyltrichlorosilane (321 ml, Aldrich Chemical Company) is added, and the reaction mixture is refluxed overnight (16 hours) with slow mechanical stirring at about 60°C. Care is taken so that the stirrer does not reach near the bottom of the flask. This is to prevent grinding the silica gel particles.

The mixture is allowed to cool. The silanized silica gel is collected, washed with toluene (3 L) and

acetone (3 L), and then air-dried overnight (16-20 hours). The dried silica gel is suspended in 3.5 L of acetonitrile:water (1:1) in a 5-L flask, stirred carefully at room temperature for 2 hours, filtered, washed with acetone (3 L) and air-dried overnight.

Step 3: Second Silylation

The procedure from the first silylation is repeated using 200 ml of octadecyltrichlorosilane. The suspension is refluxed at 60°C. for 2 hours while stirring carefully. The final product is recovered by filtration, washed with toluene (3 L) and methanol (6 L), and then dried under vacuum at 50°C. overnight (16-20 hours).

## Reference Example 3

Slurry Packing Procedure for Michel-Miller Columns

General Information

A. Analytical or preparative columns can be packed by this procedure.

B. Silica gels (e.g., Quantum LP-1, particle size 10-20 microns) and silica gel reversed phase packings (e.g., LiChroprep RP-8 and RP-18, particle size 25-40 microns) are recommended. However, other silica gels (e.g., Shandons ODS Hypersil, particle size 5 microns) as well as other types of resins have been packed successfully by this procedure.

C. Generally, a pressure of less than 200 psi and flow rates between 5-40 ml/minute are required for this slurry packing technique; this is dependent on

column volume and size. Packing pressure should exceed pressure used during actual separation by 30-50 psi; this will assure no further compression of the adsorbent during separation runs. Columns packed by this procedure with reversed-phase silica gel can be operated for several years without loss of efficiency.

D. Sudden decrease in pressure may cause cracks or channels to form in the packing material, which would greatly reduce column efficiency. Therefore, it is important to let the pressure drop slowly to zero whenever the pump has been turned off.

E. Approximate volume of columns (Ace Glass Cat. No., unpacked): 5795-04, 12 ml; 5795-10, 110 ml; 5795-16, 300 ml; 5795-24, 635 ml; and 5796-34, 34 ml.

F. The time required to pack a glass column will vary from minutes to several hours depending on column size and experience of the scientist.

<u>Procedure:</u>

1. Connect glass column to a reservoir column via coupling (volume of reservoir column should be twice that of the column). Place both columns in vertical positions (reservoir column above).

2. Weigh out packing material (<u>ca</u>. 100 g for 200 ml column).

3. Add <u>ca</u>. five volumes of solvent to packing material; use a mixture of 70-80% methanol and 20-30% water.

4. Shake well until all particles are wetted, let stand overnight or longer to assure complete soaking of particles by solvent. Decant supernatant.

5. Slurry the resin with sufficient solvent to fill reservoir column. Pour swiftly into reservoir. The column must be pre-filled with the same solvent and the reservoir column should be partly filled with solvent before slurry is poured. The use of larger slurry volumes may also provide good results; however, this will require (a) larger reservoir or (b) multiple reservoir fillings during the packing procedure.

6. Close reservoir with the Teflon plug beneath the column (see Figure 1 of U.S. Patent 4,131,547, plug No. 3); connect to pump; and immediately start pumping solvent through system at maximum flow rate if Ace Cat. No. 13265-25 Pump or similar solvent delivery system is used (ca. 20 ml/minute).

7. Continue until column is completely filled with adsorbent. Pressure should not exceed maximum tolerance of column during this operation (ca. 200 psi for large columns and 300 psi for analytical columns). In most cases, pressures less than 200 psi will be sufficient.

8. Should pressure exceed maximum values, reduce flow-rate; pressure will drop.

9. After column has been filled with adsorbent, turn off pump; let pressure drop to zero; disconnect reservoir; replace reservoir with a pre-column; fill pre-column with solvent and small amount of adsorbent; and pump at maximum pressure until column is completely packed. Always allow pressure to decrease slowly after turning off pump--this will prevent formation of any cracks or channels in the packing material.

10. Relieve pressure and disconnect pre-column carefully. With small spatula remove a few mm (2-4) of packing from top of column; place 1 or more filter(s) on top of column; gently depress to top of packing material, and place Teflon plug on top of column until seal is confirmed. Connect column to pump, put pressure on (usually less than 200 psi) and observe through glass wall on top of column if resin is packing any further. If packing material should continue to settle (this may be the case with larger columns), some dead space or channelling will appear and step 9 should be repeated.

Reference Example 4

Aculeacin Aα, Aγ, Dα, and Dγ are prepared by the following procedure:

One hundred ml. of an aqueous medium (pH 6.5) containing 2% glucose, 1% polypeptone, 1% maize steep liquor, 0.2% $KH_2PO_4$ and 0.1% $MgSO_4 \cdot 7H_2O$ is sterilized at 120°C. for 15 minutes, inoculated with NRRL 11270 and subjected to shaken culture at 26°C. for 48 hours. The obtained culture is used to inoculate 15 L of an aqueous medium (pH 6.5) containing 1.5% sucrose, 1.4% dextrin, 2% polypeptone, 0.45% maize steep liquor, 0.2% $KH_2PO_4$, 0.1% $MgSO_4$ and an antifoam. Culture is effected at 26°C. for 96 hours with aeration at 30 L/min. and stirring at 25 rpm.

The obtained broth (10 L) is filtered to give 1 kg of wet mycelium. This is extracted twice with methanol (5 L). The combined extracts are evaporated

to give 2 L of a concentrate which is diluted with water (2 L) and extracted twice with n-butanol (2 L). The butanol extract is washed with 5 L of water and concentrated while adding a small amount of water to give a viscous concentrate. This concentrate is treated with n-hexane (500 ml). The precipitate obtained is washed (ethyl acetate) to give 12.5 g of crude aculeacins. This material is purified by reversed phase $C_{18}$ silica gel HPLPLC using the procedure described in Example 1 and solvents appropriate for the aculeacin factors.

Reference Example 5

Antibiotic S 31794/F-1 is prepared by the following procedure: submerged fermentation of Acrophialophora limonispora NRRL 8095 with stirring, shaking, and/or aeration at pH of from 3 to 8, preferably at pH of from 5 to 7, and at a temperature of from 15 to 30°C., preferably from 18 to 27°C. for 48 to 360 hours (preferably 120 to 288 hours).

Culture broth prepared in this manner (90 L) is treated with ethyl acetate:isopropanol (4:1, 90 L) and homogenized 30 minutes at room temperature. The organic phase is separated and evaporated in vacuo at 40°C. The residue is chromatographed on a 10-fold amount of silica gel using $CHCl_3$:MeOH (95:5 to 60:40).

Antifungally active eluate fractions are combined and chromatographed on a 100-fold amount of "Sephadex LH-20" (RTM) with methanol. The antifungally active eluate fractions are combined.

Example 1

Isolation of A-30912 Factor A

A-42355 antibiotic mixture (1 g), prepared as described in Section D, Reference Example 1, was dissolved in 7 ml of methanol:water:acetonitrile (7:2:1). This solution was filtered and introduced onto a 3.7-cm I.D. x 35-cm glass column [Michel-Miller High Performance Low Pressure (HPLPLC) Chromatography Column, Ace Glass Incorporated, Vineland, NJ 08360] packed with LP-1/$C_{18}$ silica gel reversed-phase resin (10-20 microns), prepared as described in Reference Example 2, through a loop with the aid of a valve system. The column was packed in methanol:water:acetonitrile (7:2:1) by the slurry-packing procedure described in Reference Example 3. An F.M.I. pump with valveless piston design (maximum flow 19.5 ml/minute) was used to move the solvent through the column at a flow rate of 9 ml/minute at ca. 100 psi, collecting fractions every minute. Elution of the antibiotic was monitored at 280 nm by using a UV monitor (ISCO Model UA-5, Instrument Specialist Co., 4700 Superior Ave., Lincoln, Nebraska 68504) with an optical unit (ISCO Type 6).

Fractions 112-140 were combined and added to 20 ml of water. The pH of this solution was adjusted to pH 4.0 with N HCl. The resulting solution was extracted twice with equal volumes of chloroform. The two chloroform extracts were combined and concentrated under vacuum to give an oil. The oil was dissolved in tertiary butanol, and this solution was lyophilized to give 524 mg of A-42355 factor A (A-30912 factor A; A-22082).

Example 2

Isolation of A-30912 Factor B

A-42355 mixture was separated as described in Reference Example 1 except that the concentrated chloroform extracts (285 L) were chromatographed over a silica-gel column (150 L of Grace silica-gel, grade 62) at a flow rate of 2 L/min. The column was washed with chloroform (200 L), eluted with acetonitrile (500 L), and then continuously eluted with acetonitrile:water (98:2) at a flow rate of 1 L/min. Fractions having a volume of approximately 200 L were collected and analyzed individually for biological activity. The bioassay was performed by a paper-disc assay on agar plates seeded with *Candida albicans*. Fractions 77 through 103 (1365 L) were combined and concentrated under vacuum. The concentrated solution (4.5 L) contained a precipitate which was removed by filtration to give 119 g of factor B-enriched A-42355 mixture. The filtrate was concentrated to dryness; the residue obtained was redissolved in an appropriate volume of methanol. The methanol solution was added to diethyl ether (10 volumes) to precipitate the factor-B-containing antibiotic mixture. This precipitate was also separated by filtration and dried to give an additional 24 g of factor-B-enriched A-42355 mixture as a gray powder.

Factor-B-enriched A-42355 mixture thus obtained (1.0 g) was dissolved in 8 ml of methanol:water:acetonitrile (7:2:1). This solution was filtered and introduced onto a silica-gel column (3.7-cm I.D. x

33-cm Michel-Miller Column) through a loop with the aid of a valve system. The column was packed with LP-1/$C_{18}$ silica-gel reversed-phase resin (10-20 microns, prepared as described in Reference Example 2) in methanol:-water:acetonitrile (7:2:1) as described in Example 1. The solvent was moved through the column at a flow rate of 10 ml/min at ca. 100 psi as in Example 1. One fraction was collected every minute. Elution of the antibiotic was monitored at 280 nm as in Example 1. Fractions 102-110 were combined and concentrated under vacuum to give an oil. The oil was dissolved in a small volume of tert-butanol and lyophilized to give 22 mg of A-30912 factor B.

## Example 3

### Isolation of A-30912 Factor D

Concentrated chloroform extracts from two fermentation runs (3800 L and 4007 L) obtained by the method described in Reference Example 1 were combined and chromatographed on a silica-gel column as described in Example 2. Fractions having a volume of approximately 200 L were collected and subjected to bioassay as in Example 2. Fractions 47-63 (850 L) were combined and concentrated under vacuum. The concentrated solution (0.7 L) was added to diethyl ether (10 volumes) to precipitate the factor D-enriched A-42355 mixture. This precipitate was removed by filtration and dried to give 32 g of factor D-enriched A-42355 mixture as a gray powder.

Factor D-enriched A-42355 mixture thus obtained (1.0 g) was dissolved in 5 ml of methanol: water:acetonitrile (7:2:1). This solution was filtered and introduced onto a silica-gel column (3.7-cm I.D. x 30-cm Michel-Miller Column) through a loop with the aid of a valve system. The column was packed with LP-1/$C_{18}$ silica-gel reversed-phase resin (10-20 microns, prepared as described in Reference Example 2). Packing was accomplished in methanol:water:acetonitrile (7:2:1) as described in Example 1. The solvent was moved through the column at a flow rate of 8 ml/min at ca. 45 psi as described in Example 1. One fraction was collected every 2 minutes. Elution of the antibiotic was monitored at 280 nm as described in Example 1. Fractions 96-108 were combined and concentrated under vacuum to give an oil. This oil was dissolved in a small volume of tert-butanol and lyophilized to give 89 mg of A-30912 factor D.

## Example 4

### Isolation of A-30912 Factor H

A-42355 antibiotic mixture (5.0 g), prepared as described in Reference Example 1, was dissolved in 35 ml of methanol:water:acetonitrile (7:2:1); the resulting solution was filtered and introduced onto a 3.7-cm I.D. x 42-cm glass column (Michel-Miller Column) through a loop with the aid of a valve system. The column was packed with LP-1/$C_{18}$ silica gel reversed phase resin (10-20 microns, prepared as described in Example 2) in methanol:water:acetonitrile (7:2:1) as

described in Example 1. The solvent was moved through the column at a flow rate of 13 ml/min at ca. 120 psi as described in Example 1, collecting one fraction every two minutes. Elution of the antibiotic was monitored at 280 nm as described in Example 1. Fractions 112-132 were combined with fractions 106-117 from a second similar purification. The combined fractions were concentrated under vacuum to an oil. The oil was dissolved in a small volume of tert-butanol and lyophilized to give 173 mg of crude A-30912 factor H.

The crude A-30912 factor H (150 mg) was dissolved in 8 ml of methanol:water:acetonitrile (7:2:1); the resulting solution was filtered and introduced onto a 2.0-cm I.D. x 32-cm glass column, as described above. The solvent was moved through the column at a flow rate of 8 ml/min at ca. 80 psi as described in Example 1, collecting one fraction every three minutes. Elution of the antibiotic was monitored at 280 nm as described in Example 1. Fractions 17 and 18 were combined and concentrated under vacuum to give an oil. The oil was dissolved in a small volume of tert-butanol and lyophilized to give 29 mg of A-30912 factor H.

## Example 5

Aculeacin factors A, B, C, D, E, F and G are prepared as described in U.S. Patent 3,978,210 by fermentation of *Aspergillus aculeatus* M 4214, NRRL 8075, and are then purified by reversed-phase $C_{18}$-silica-gel HPLPLC, using the procedure described in Example 1 but using solvents appropriate for the aculeacin factors.

Example 6

Antibiotic S 31794/F-1 is prepared as described in Reference Example 5 and then purified by reversed-phase $C_{18}$-silica-gel HPLPLC using the procedure described in Example 1 and solvents appropriate for antibiotic S 31794/F-1.

## CLAIMS

1. A process for recovering an A-30912-type antibiotic selected from the group consisting of A-30912 factors A, B, C, D, and H; the tetrahydro-derivatives of A-30912 factors A, B, C, D, and H; aculeacin factors A, Aα, Aγ, B, C, D, Dα, Dγ, E, F and G; and antibiotic S 31794/F-1; which comprises a) contacting a solution of said antibiotic with a reversed-phase high-loading-capacity silica gel, b) carrying out liquid chromatography and c) recovering the antibiotic.

2. The process of claim 1 wherein the chromatography is high performance liquid chromatography.

3. The process of claim 2 wherein the chromatography is high performance, low pressure liquid chromatography.

4. The process of claim 1 wherein the reversed-phase resin is a high carbon content resin, the carbon content being at least 15 per cent.

MICRONS
2.5
3
4
5
6
7
8
9
10
12
14
16 18 20
25 30 40
PERCENT TRANSMISSION
100
80
60
40
20
0
WAVENUMBER (CM⁻¹)
4000
3500
3000
2500
2000
1800
1600
1400
1200
1000
800
600
400
250

*FIG. 1*

D
C
H
B
A
EFG
+

FIG. 2

0030844

European Patent Office

# EUROPEAN SEARCH REPORT

Application number
EP 80 30 4472

## DOCUMENTS CONSIDERED TO BE RELEVANT

| Category | Citation of document with indication, where appropriate, of relevant passages | Relevant to claim |
|---|---|---|
| X | EP - A - 0 005 956 (ELI LILLY)<br>* Page 39, lines 12-15 * | 1-4 |
| D | US - A - 4 024 245 (ELI LILLY)<br>* In its entirety * | 1-4 |
| D | US - A - 3 978 210 (ELI LILLY)<br>* In its entirety * | 1-4 |

CLASSIFICATION OF THE APPLICATION (Int. Cl.3)

C 07 C 103/52
C 12 P 21/04
(C 21/04
C 12 R 1/66)

TECHNICAL FIELDS SEARCHED (Int. Cl.3)

C 07 C 103/52
C 12 P 21/00
21/04
C 12 R 1/06
1/66

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | March, 1981 | GRAMAGLIA |

EPO Form 1503.1 06.78